# EUROPEAN PATENT SPECIFICATION

(11) **EP 2 729 117 B1**
(45) Date of publication and mention of the grant of the patent: **27.02.2019**
(21) Application number: 12731473.0
(22) Date of filing: 05.07.2012
(51) Int. Cl.: A61K 8/49, A61Q 17/04, A61P 17/16, A61P 17/18

(54) **PHOTOPROTECTIVE COMPOSITION**
LICHTSCHUTZZUSAMMENSETZUNG
COMPOSITION PHOTOPROTECTRICE

(30) Priority: 07.07.2011 FR 1156177; 19.07.2011 US 201161509338 P
(43) Date of publication of application: 14.05.2014
(73) Proprietor: L'OREAL, 75008 Paris (FR)
(72) Inventor: JOUY, Chantal, F-75005 Paris (FR); RUIZ, Laëtitia, F-94430 Chennevieres-sur-Marne (FR); PYGMALION, Marie-Jocelyne, F-94220 Charenton Le Pont (FR)
(74) Representative: Lavoix
(86) International application number: PCT/EP2012/063114
(87) International publication number: WO 2013/004777

(56) References cited:
- WO-A1-2008/140440
- DE-A1- 19 755 504
- DE-A1- 19 923 713
- DE-A1- 19 923 715
- DE-A1-102009 037 900
- JP-A- 2011 093 858
- ZHOU BING-RONG ET AL: "Protective effect of baicalin against multiple ultraviolet B exposure-mediated injuries in C57BL/6 mouse skin.", ARCHIVES OF PHARMACAL RESEARCH FEB 2011 LNKD- PUBMED:21380810, vol. 34, no. 2, February 2011 (2011-02), pages 261-268, XP002670690, ISSN: 0253-6269
- DATABASE WPI Week 200953 Thomson Scientific, London, GB; AN 2009-L39356 XP002670691, & CN 101 467 938 A (TIANJIN TIANFUKANG BIO TECHNOLOGY DEV CO LTD) 1 July 2009 (2009-07-01)
- PARK S-J ET AL: "Rapid in vivo screening system for anti-oxidant activity using bacterial redox sensor strains", JOURNAL OF APPLIED MICROBIOLOGY, vol. 108, no. 4, April 2010 (2010-04), pages 1217-1225, XP002670692, ISSN: 1364-5072
- DATABASE WPI Week 198823 Thomson Scientific, London, GB; AN 1988-157190 XP002670693, & JP 63 096120 A (MATSUURA YAKUGYO KK) 27 April 1988 (1988-04-27)

## Description

The present invention relates to a composition for use in a method of photoprotection comprising topical application of said composition comprising an association of baicalin or an extract containing same with at least one system filtering UVA radiation. The invention particularly relates to the field of cosmetics and/or beauty products.

The present invention also relates to the use of baicalin, or an extract containing same, in association with at least one system filtering UVA radiation comprising at least one bis-resorcinyl triazine for protecting the skin and/or lips and/or hair against solar radiation.

It is known that UV-A rays, having wavelengths between 320 and 400 nm, which cause the skin to tan, are liable to induce damage thereof, particularly in the case of sensitive skin or skin continually exposed to solar radiation. UV-A rays particular cause a loss of skin elasticity and the appearance of wrinkles giving rise to premature skin ageing. In this way, for esthetic and cosmetic reasons, such as preserving natural skin elasticity, for example, an increasing number of people are seeking to control the effect of UV-A rays on their skin. It is thus desirable to filter UV-A radiation.

It is also known that light radiation with wavelengths between 280 nm and 400 nm enables tanning of the human epidermis, and that rays having wavelengths more specifically between 280 and 320 nm, known as UV-B, impede the development of a natural tan. For these reasons and for esthetic reasons, there is a constant demand for means for controlling natural tanning with a view to controlling skin color; it is thus necessary to filter UV-B radiation.

In order to protect the skin and keratin material against UV radiation, sunscreen compositions comprising organic filters, active in the UV-A range and active in the UV-B range, are generally used. The majority of these filters are lipophilic, although they also include hydrophilic compounds.

### State of the related art

There is scope for further enhancement of existing photoprotective compositions in respect of protecting the skin against UV radiation, particularly in respect of UVA, but also UVB, radiation.

The development of cosmetic compositions suitable for combating premature skin ageing effectively involves the use of sun filters, particularly those absorbing in the UVA range. These filters make it possible to reduce excess photoinduced free radicals. However, with the majority of compositions containing UV filters, particularly UVA filters, complete protection is not provided. In this way, following repeated exposure, the residual amount of free radicals persisting in spite of UVA filter protection may, in the long-term, cause photoactinic ageing phenomena. One solution may consist of increasing the amounts of UVA filters, but it is not recommended to use excessively high levels of filters in cosmetic products for everyday use.

Indeed, with the majority of filters, a maximum protection factor is reached, which is very difficult to improve by increasing the proportion of filters.

In this way, research on solutions, aiming to find a means other than that consisting of using large amounts of UV filters, particularly UVA filters, for use in a method protecting the skin against sun rays and combating premature skin ageing effectively under conditions of use and in concentrations acceptable for those skilled in the art, is still of interest.

In the prior art, the use of baicalin in cosmetic compositions, and particularly in photoprotective compositions, has already been suggested. This particularly stems from a number of publications and patents such as the patent application JP 63083017 relating to the filtering action of some compounds including baicalin.

The publications by Shengzhao et al. (Study of application of baicalin in functional cosmetics, Shengzhao Gong; Yuke Jie; Shuiming Yuan, Riyong Huaxue Gongye (2003), 33(3), 200-203) and Feng-jiao et al. (Technology for preparation of sunscreen cream containing baicalin, Feng-jiao Sun; Ke-jian Liao; Yu-feng Cong; Yue-ling Dai; Feng Yan, Riyong Huaxue Gongye (2009), 39(4), 257-259) also relate to cosmetic compositions, and more specifically sun protection compositions, containing baicalin.

The patent application DE102009037900 also describes a cosmetic composition comprising baicalin in association with various active ingredients. This document also describes the possible additional presence of UV filtering agents, but with no particular conclusion in this respect.

The international application WO 98/196651 describes a composition comprising mineral (non-chemical) filters in the presence of proanthocyanidins, ferulic acid oryzanol and/or an ester containing same and optionally a *scutellaria* extract. The possibility of using baicalin is mentioned; however, it is clear from the examples that the very specific association of ethyl ferulate, pycnogenol, oryzanol and scutellaria extract improves the skin protection factor (SPF). However, these photoprotective compositions comprising baicalin require further enhancement in terms of protecting the skin against solar radiation, particularly UV. In this context the document DE 19923715 relates to a composition comprising alpha-glycosylrutine.

There is thus still a need to identify compositions, particularly cosmetic compositions protecting the skin against damage induced by sun rays.

### Aims of the invention

The aim of the invention is that of solving the technical problem consisting of providing a photoprotective composition for use in a method of photoprotection comprising topical application of a composition according to claim 1.

The aim of the invention is also that of solving the technical problem consisting of improving photoprotective compositions in terms of protection of the skin and/or hair and/or lips against solar radiation, particularly UV.

A further aim of the invention is that of finding a means other than that consisting of using large amounts of UV filters, particularly UVA filters, for combating premature ageing of the skin and/or lips and/or hair effectively under conditions of use and in concentrations acceptable for those skilled in the art.

A further aim of the invention is that of preventing and/or combating premature ageing of the skin and/or lips due to ultraviolet radiation and protecting the skin and/or lips and/or hair against this form of radiation and free radicals.

The invention thus proposes to solve these various technical problems

### Description of the invention

The Applicant has now discovered, surprisingly, that, by associating baicalin, or an extract containing same, with at least one system filtering UVA radiation comprising at least one bis-resorcinyl triazine derivative, it was possible to enhance the protection of the skin and/or lips and/or hair against UV, particularly in respect of UVA radiation.

In this way, according to one of the aims of the present invention, a photoprotective composition for topical use comprising baicalin or an extract containing same, and at least one system filtering UVA radiation comprising at least one bis-resorcinyl triazine derivative. The invention particularly relates to a cosmetic composition. The system filtering UVA radiation advantageously further comprises at least one UVA filter selected from a dibenzoylmethane derivative, a compound filtering UVA derived from benzylidene camphor and a mixture of these two types of derivatives.

According to one preferred embodiment, the composition according to the invention further comprises at least one UVB filter, particularly of the β,β'-diphenylacrylate type.

The composition according to the invention more specifically covers a composition comprising baicalin, or an extract containing same; at least one system filtering UVA radiations comprising at least one bis-resorcinyl triazine derivative, at least one dibenzoylmethane derivative, and at least one compound filtering UVA derived from benzylidene camphor; and at least one UVB filter particularly of the β,β'-diphenylacrylate type, and preferably a composition comprising baicalin, or an extract containing same; and at least one system filtering UVA radiations comprising at least Bis-Ethylhexylphenol Methoxyphenyltriazine, Avobenzone, Terephthalylidene Dicamphor Sulfonic Acid; said composition further comprising octocrylene.

The composition according to the invention more specifically further comprises compounds filtering UVA and/or UVB. The composition according to the invention preferably further comprises cosmetically acceptable excipients.

The invention also relates to a multi-constituent agent for use in a method of photoprotection of human keratin material (skin, lips, hair, eyelashes, eyebrows, nails) comprising a composition A and a composition B packaged separately; said composition A comprising, in a cosmetically acceptable medium, at least baicalin or an extract;
said composition B comprising, in a cosmetically acceptable medium, at least one system filtering UVA radiation comprising at least one bis-resorcinyl triazine derivative; said composition A and said composition B being applied either simultaneously or sequentially on the surface of the keratin material.

In the case of sequential application, composition A comprising baicalin is preferably applied first. The time interval between the applications of composition A and composition B preferably varies from 1 hour to 48 hours and more preferentially from 1 hour to 24 hours. The filtering composition B could be applied immediately prior to UV and particularly solar radiation exposure.

The system filtering UVA radiation advantageously further comprises at least one UVA filter selected from a dibenzoylmethane derivative, a compound filtering UVA derived from benzylidene camphor and a mixture of these two types of derivatives.

Composition B according to the invention specifically contains at least one system filtering UVA radiation comprising at least one bis-resorcinyl triazine derivative, at least one dibenzoylmethane derivative, and at least one compound filtering UVA derived from benzylidene camphor.

According to one preferred embodiment, the composition according to the invention further comprises at least one UVB filter, particularly of the β,β'-diphenylacrylate type.

According to one preferred embodiment, composition B according to the invention comprises at least one system filtering UVA radiation comprising at least Bis-Ethylhexylphenol Methoxyphenyltriazine, Avobenzone, Terephthalylidene Dicamphor Sulfonic Acid; said composition further comprising octocrylene.

Composition B according to the invention more specifically further comprises compounds filtering UVA and/or UVB. The composition according to the invention preferably further comprises cosmetically acceptable excipients.

A further aim of the invention relates to the use of baicalin, or an extract containing same, in association with at least one system filtering UVA radiation comprising at least one bis-resorcinyl triazine in a method of for protecting the skin and/or lips and/or hair against solar radiation, and/or for combating or preventing photoinduced premature ageing of the skin and/or lips and/or hair.

The present invention also relates to a use of a composition or a multi-constituent agent according to the invention, in a method for combating or preventing photoinduced premature ageing of human keratin material (skin, lips, hair, eyelashes, eyebrows, nails).

The present invention also relates to a use comprising the topical application on human keratin material (skin, lips, hair, eyelashes, eyebrows, nails) of a composition or a multi-constituent agent as defined, in a method for protecting the human keratin material (skin, lips, hair, eyelashes, eyebrows, nails) against solar radiation.

Further features, aspects and advantages of the invention will emerge on reading the detailed description hereinafter.

### Definitions

The term system filtering UV (UVA and/or UVB) radiation refers to a substance capable of absorbing at least a portion of UV (UVA and/or UVB) radiation emitted by the sun for protecting the skin and/or lips and/or hair against the harmful effects of this radiation. UVA radiation wavelengths are different to UVB radiation wavelengths. UVA radiation wavelengths are between 400 nm and 315 nm and for UVB between 315 nm and 280 nm.

The term "associate" compounds or "association" of compounds refers to the presence, in the same composition, or in separate compositions applied sequentially or simultaneously, of the compounds to which reference is made.

The term "cosmetically acceptable" means compatible with the skin and/or integuments thereof, having a pleasant color, odor and texture and not giving rise to unacceptable discomfort (tingling, tightness, redness), liable to dissuade the consumer from using the composition.

The compositions according to the invention, particularly compositions A and/or B, may additionally comprise further complementary organic or inorganic UV filters active in the UVA and/or UVB range, which are hydrophilic or lipophilic or insoluble in routine cosmetic solvents.

The term "lipophilic UV filter" refers to any cosmetic or dermatological filter liable to be completely dissolved in the molecular state in a liquid fat phase or be solubilized in colloidal form (for example in micellar form) in a liquid fat phase.

The term "hydrophilic UV filter" refers to any cosmetic or dermatological filter liable to be completely dissolved in the molecular state in a liquid aqueous phase or be solubilized in colloidal form (for example in micellar form) in a liquid aqueous phase.

The term "insoluble UV filter" refers to any cosmetic or dermatological filter which is not defined as a lipophilic UV filter or as a hydrophilic UV filter, in particle form in liquid aqueous or fat phase.

### Detailed description of the invention

The invention is detailed hereinafter with reference to the more general description of the invention.

Baicalin has the following formula:

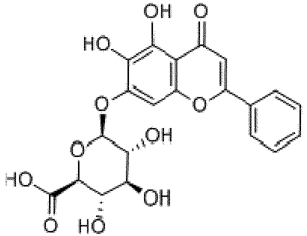

Baicalin is available from MMP or Guilin Layn Natural Ingredients Corp. for example. This compound is particularly described in the application WO 2008/140440 particularly in stable solution form. Baicalin may be used in stable solution form comprising a glycol alkyl having 2 to 7 carbon atoms, a polyol ether, and at least one anti-oxidant.

Such an organic compound may be obtained as described in EP 1400 579 (US2004/0067894) relating to the synthesis of tetrahydroxyflavones wherein the general formula comprises baicalin.

Baicalin may be used in the form of an extract of plant origin. Baicalin is a polyphenol (flavone) particularly extracted from skull cap root (*scutellaria* and particularly *scutellaria baicalensis*), based on traditional Chinese medicine.

The bis-resorcinyl triazine compounds according to the present invention preferably comply with the following formula (I): 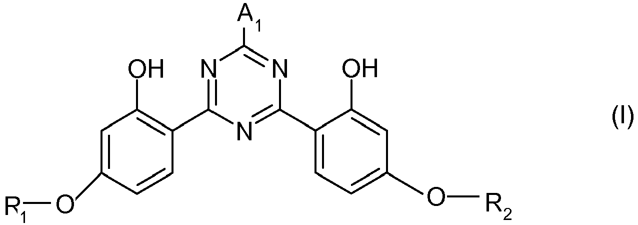 in which:
(i) the radicals R₁ and R₂, which are identical or different, refer to a C₃-C₁₈ alkyl radical; a C₂-C₁₈ alkenyl radical or a residue having the formula -CH₂CH(OH)-CH₂OT₁ where T₁ is a hydrogen atom or a C₁-C₈ alkyl radical;
(ii) the radicals R₁ and R₂, which are identical or different, may also refer to a residue having the following formula (1): 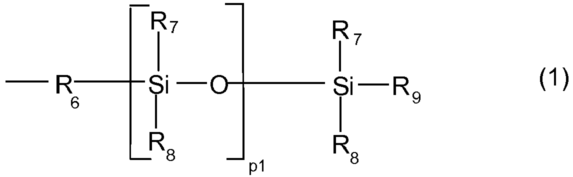 in which:
   - R₆ refers to a covalent bond; a C₁-C₄ linear or branched alkyl radical or a residue having the formula -Cₘ₁H₂ₘ₁-O- where m₁ is a number from 1 to 4;
   - p₁ is a number from 0 to 5;
   - the radicals R₇, R₈ et R₉, which are identical or different, refer to a C₁-C₁₈ alkyl radical; a C₁-C₁₈ alkoxy radical or a residue having the formula: 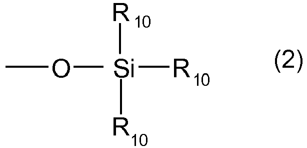
   where R₁₀ is a C₁-C₅ alkyl radical;
   - A₁ refers to a residue complying with any of the following formulas: 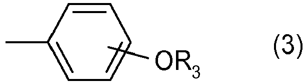 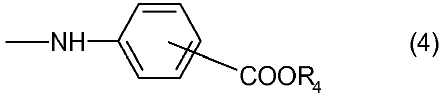 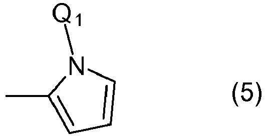
   in which:
   - R₃ refers to a hydrogen atom, a C₁-C₁₀ alkyl radical, a radical having the formula:
   - (CH₂CHR₅-O)ₙ₁R₄ where n₁ is a number from 1 to 16, or a residue having the structure
   - CH₂CH-(OH)-CH₂OT₁ where T₁ has the same meaning as mentioned above.
   - R₄ refers to hydrogen, a metal cation M, a C₁-C₅ alkyl radical or a residue having the formula -(CH₂)m₂OT₁ where m₂ is a number from 1 to 4 and T₁ has the same meaning as mentioned above.
   - Q₁ is a C₁-C₁₈ alkyl radical.

In formulas (I) and (1) to (5) described above:
- the alkyl radicals are linear or branched and may be selected for example from methyl, ethyl, n-propyl, isopropyl, n-butyl, sec.butyl, tert.butyl, amyl, isoamyl, tert.amyl, heptyl, octyl, isooctyl, nonyl, decyl, undecyl, dodecyl, tetradecyl, pentadecyl, hexadecyl, heptadecyl or octadecyl;
- the alkenyl radicals may be selected for example from allyl, methallyl, isopropenyl, 2-butenyl, 3-butenyl, isobutenyl, n-penta-2,4-dienyl, 3-methyl-but-2-enyl, n-oct-2-enyl, n-dodec-2-enyl, iso-dodecenyl, n-octadec-4-enyl;
- the alkoxy radicals are linear or branched and may be selected for example from methoxy, ethoxy, n-propoxy, isopropoxy, n-butoxy, sec.-butoxy, tert.-butoxy, amyloxy, isoamyloxy or tert.amyloxy;
- the C₁-C₅ mono or dialkylamino radicals may be selected for example from methylamino, ethylamino, propylamino, n-butylamino, sec.butylamino, tert.butylamino, pentylamino, dimethylamino, diethylamino, dibutylamino or methylethylamino.
- the metallic cations are alkaline, alkaline-earth or metallic cations selected for example from lithium, potassium, sodium, calcium, magnesium, copper and zinc.

The bis-resorcinyl triazine derivatives having formula (I) according to the invention are filters already known per se. They are described and prepared according to the synthesis processes given in the patent applications EP-A-0775 698.

Examples of compounds having formula (I) suitable for use include:
- 2,4-bis{[4-(2-ethyl-hexyloxy)-2-hydroxy]-phenyl}-6-(4-methoxy-phenyl)-1,3,5-triazine;
- 2,4-bis{[4-(3-(2-propyloxy)-2-hydroxy-propyloxy)-2-hydroxy)-2-phenyl}-6-(4-methoxyphenyl)-1,3,5-triazine;
- 2,4-bis{[4-(2-ethyl-hexyloxy)-2-hydroxy]-phenyl}-6-[4-(2-methoxyethyl-carboxyl)-phenylamino]-1,3,5-triazine;
- 2,4-bis{[4-tris(trimethylsiloxy-silylpropyloxy)-2-hydroxy]-phenyl}-6-(4-methoxyphenyl)-1,3,5-triazine;
- 2,4-bis{[4-(2"-methylpropenyloxy)-2-hydroxy]-phenyl}-6-(4-methoxyphenyl)-1,3,5-triazine;
- 2,4-bis{[4-(1',1',1',3',5',5',5'-heptamethyltrisiloxy-2"-methylpropyloxy)-2-hydroxy]-phenyl}-6-(4-methoxyphenyl)-1,3,5-triazine.
- 2,4-bis{[4-(3-(2-propyloxy)-2-hydroxy-propyloxy]-2-hydroxy]-phenyl}-6-[(4-ethylcarboxyl)-phenylamino]-1,3,5-triazine;
- 2,4-bis{[4-(2-ethyl-hexyloxy)-2-hydroxy]-phenyl}-6-(1-methylpyrrol-2-yl)-1,3,5-triazine.

More specifically, at least the compound 2,4-bis{[4-(2-ethyl-hexyloxy)-2-hydroxy]-phenyl}-6-(4-methoxy-phenyl)-1,3,5-triazine or Bis-Ethylhexyloxyphenol Methoxyphenyl Triazine (INCI name) such as the product sold under the trade name "TINOSORB S" by CIBA GEIGY.

The bis-resorcinyl triazine compound(s) having formula (I) are generally present in the filtering compositions according to the invention at a concentration ranging from 0.1 to 20% by weight and more preferentially from 1 to 10% by weight and more specifically from 2 to 8% by weight with respect to the total weight of the composition.

The dibenzoylmethane derivatives particularly include, but are not limited to:
- 2-methyldibenzoylmethane,
- 4-methyldibenzoylmethane,
- 4-isopropyldibenzoylmethane,
- 4-tert.-butyldibenzoylmethane,
- 2,4-dimethyldibenzoylmethane,
- 2,5-dimethyldibenzoylmethane,
- 4,4'-diisopropyldibenzoylmethane,
- 4,4'-dimethoxydibenzoylmethane,
- 4-tert.-butyl-4'-methoxydibenzoylmethane,
- 2-methyl-5-isopropyl-4'-methoxydibenzoylmethane,
- 2-methyl-5-tert-butyl-4'-methoxydibenzoylmethane,
- 2,4-dimethyl-4'-methoxydibenzoylmethane,
- 2,6-dimethyl-4-tert-butyl-4'-methoxydibenzoylmethane.

Of the dibenzoylmethane derivatives mentioned above, 4-isopropyl-dibenzoylmethane, sold under the trade name "EUSOLEX 8020" by MERCK, and complying with the formula below is used for example:

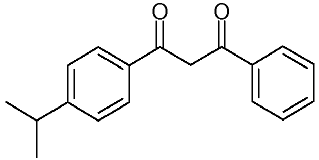

Particular preference is placed on the use of at least 4-(ter.-butyl) 4'-methoxy dibenzoylmethane or Butyl Methoxy Dibenzoylmethane or avobenzone, marketed under the trade name "PARSOL 1789" by DSM Nutritional Products, Inc; this filter complies with the following formula:

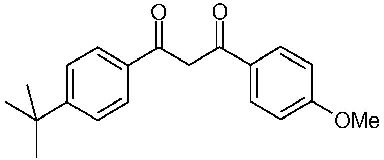

The dibenzoylmethane derivative(s) may be present in the compositions according to the invention at contents preferably varying from 0.01 to 10% by weight and more preferentially from 0.1 to 6% by weight with respect to the total weight of the composition.

The benzylidene camphor derivatives include:
3-Benzylidene camphor manufactured under the name "MEXORYL SD" by CHIMEX,
4-Methylbenzylidene camphor sold under the name "EUSOLEX 6300" by MERCK,
Benzylidene Camphor Sulfonic Acid manufactured under the name "MEXORYL SL" by CHIMEX,
Camphor Benzalkonium Methosulfate manufactured under the name "MEXORYL SO" by CHIMEX,
Polyacrylamidomethyl Benzylidene Camphor manufactured under the name "MEXORYL SW" by CHIMEX.

The use of at least Terephthalylidene Dicamphor Sulfonic Acid marketed under the name MEXORYL SX by CHIMEX is preferred.

The invention may further comprise at least one UVB filter and particular at least one β, β'-diphenylacrylate derivative. Lipophilic UVB filters are preferred.

The β,β'-diphenylacrylate derivatives include etocrylene, particularly sold under the trade name "UVINUL N35" by BASF. The use of at least octocrylene as a UVB filter is preferred. Octocrylene is particularly sold under the trade name "UVINUL N539" by BASF.

The compositions according to the invention may additional comprise complementary organic or inorganic photoprotective agents, also known as filtering agents or filters, filtering wavelengths within the UVA and/or UVB ranges, which are hydrophilic or lipophilic or insoluble in routine cosmetic solvents.

The complementary organic filters are particularly selected from anthranilics; cinnamic derivatives; salicylic derivatives; benzophenone derivatives; phenyl benzotriazole derivatives; benzalmalonate derivatives particularly those cited in the patent US5624663; phenyl benzimidazole derivatives; imidazolines; 4,4-diarylbutadiene derivatives; bis-benzoazolyle derivatives as described in the patents EP669323 and US 2,463,264; p-aminobenzoic acid (PABA) derivatives; methylene bis-(hydroxyphenyl benzotriazole) derivatives as described in the applications US5,237,071, US 5,166,355, GB2303549, DE 197 26 184 and EP893119; benzoxazole derivatives as described in the patent applications EP0832642, EP1027883, EP1300137 and DE10162844; filter polymers and filter silicones such as those particularly described in the application WO-93/04665; dimers derived from α-alkylstyrene such as those described in the patent application DE19855649; 4,4-diarylbutadienes as described in the applications EP0967200, DE19746654, DE19755649, EP-A-1008586, EP1133980 and EP133981; merocyanine derivatives such as those described in WO04006878, WO05058269 and WO06032741 and the mixtures thereof.

Examples of organic UV filters include those referred to hereinafter using the INCI name thereof:

### Classification according to UVA and/or UVB radiation wavelength range

### I/ Lipophilic UV-A filters

### Dibenzovlmethane derivatives:

Isopropyl Dibenzoylmethane;

### Aminobenzophenones:

n-hexyl 2-(4-diethylamino-2-hydroxybenzoyl)-benzoate particularly sold under the trade name "UVINUL A +" by BASF;
1,1'-(1,4-piperazinediyl)bis[1-[2-[4-(diethylamino)-2-hydroxybenzoyl]phenyl]-methanone (CAS 919803-06-8)

### Anthranilic derivatives:

Menthyl anthranilate particularly sold under the trade name "NEO HELIOPAN MA" by SYMRISE;

### 4,4-diarylbutadiene derivatives:

1,1-dicarboxy (2,2'-dimethyl-propyl)-4,4-diphenylbutadiene;

### Merocyanine derivatives:

Octyl-5-N,N-diethylamino-2-phenysulfonyl-2,4-pentadienoate;

Within the scope of the invention, and according to one particular embodiment, the following lipophilic filters are used:
Butyl Methoxydibenzoylmethane

### II/ Hydrophilic UV-A filters

The bis-benzoazolyl derivatives as described in the patents EP 669 323, and US 2,463,264 and more specifically the compound Disodium Phenyl Dibenzimidazo tetra-sulfonate sold under the trade name "NEO HELIOPAN AP" by SYMRISE;

The preferred hydrophilic UVA filter is Terephthalylidene Dicamphor Sulfonic Acid.

### III/ Lipophilic UV-B filters

Para-aminobenzoates:
Ethyl PABA;
Ethyl Dihydroxypropyl PABA;
Ethylhexyl Dimethyl PABA (ESCALOL 507 from ISP);

### Salicylic derivatives:

Homosalate particularly sold under the name "Eusolex HMS" by Rona/EM Industries;
Ethylhexyl Salicylate particularly sold under the name "NEO HELIOPAN OS" by SYMRISE;
Dipropyleneglycol Salicylate particularly sold under the name "DIPSAL" by SCHER; TEA Salicylate sold under the name "NEO HELIOPAN TS" by SYMRISE;

### Cinnamates

Ethylhexyl Methoxycinnamate particularly sold under the trade name "PARSOL MCX" by DSM Nutritional Products, Inc.;
Isopropyl Methoxy cinnamate;
Isoamyl Methoxy cinnamate particularly sold under the trade name "NEO HELIOPAN E 1000" by SYMRISE;
Diisopropyl Methylcinnamate;
Cinnoxate;
Glyceryl Ethylhexanoate Dimethoxycinnamate;

### β,β'-diphenylacrylate derivatives:

Etocrylene, particularly sold under the trade name "UVINUL N35" by BASF;

### Benzylidene camphor derivatives:

3-Benzylidene camphor manufactured under the name "MEXORYL SD" by CHIMEX;
Methylbenzylidene camphor sold under the name "EUSOLEX 6300" by MERCK;
Polyacrylamidomethyl Benzylidene Camphor manufactured under the name "MEXORYL SW" by CHIMEX;

### Triazine derivatives:

Ethylhexyl triazone particularly sold under the trade name "UVINUL T150" by BASF;
Diethylhexyl Butamido Triazone particularly sold under the trade name "UVASORB HEB" by SIGMA 3V;
2,4,6-tris(dineopentyl 4'-amino benzalmalonate)-s-triazine;
2,4,6-tris(diisobutyl 4'-amino benzalmalonate)-s- triazine;
2,4-bis(dineopentyl 4'-amino benzalmalonate)-6-(n-butyl 4'-aminobenzoate)-s-triazine;
2,4-bis(n-butyl 4'-amino benzoate)-6-(aminopropyltrisiloxane)-s-triazine;
the symmetrical triazine filters described in the patent US 6,225,467, the application WO 2004/085412 (see compounds 6 and 9) or the document "Symmetrical Triazine Derivatives" IP.COM Journal, IP.COM INC WEST HENRIETTA, NY, US (September 20, 2004) particularly 2,4,6-tris-(biphenyl)-1,3,5-triazine (particularly 2,4,6-tris(biphenyl-4-yl-1,3,5-triazine) and 2,4,6-tris(terphenyl)-1,3,5-triazine, the latter two filters being described in the BEIERSDORF applications WO 06/035000, WO 06/034982, WO 06/034991, WO 06/035007, WO 2006/034992, WO 2006/034985).

### Imidazoline derivatives:

Ethylhexyl Dimethoxybenzylidene Dioxoimidazoline Propionate,

### Benzalmalonate derivatives:

Polyorganosiloxanes with a benzalmalonate function such as Polysilicone-15 particularly sold under the trade name "PARSOL SLX" by DSM Nutritional Products, Inc.;
Di-neopentyl 4'-methoxybenzalmalonate;

Within the scope of the invention, and according to one particular embodiment, the following lipophilic UV-B filters are used in the composition according to the invention: Ethylhexylsalicylate;
Octocrylene;
Ethylhexyl triazone.

### IV/ Hydrophilic UV-B filters

The following p-aminobenzoic acid (PABA) derivatives:
PABA,
Glyceryl PABA and
PEG-25 PABA particularly sold under the trade name "UVINUL P25" by BASF. Phenylbenzimidazole Sulfonic Acid particularly sold under the trade name "EUSOLEX 232" by MERCK,
ferulic acid,
salicylic acid,
DEA methoxycinnamate,
Benzylidene Camphor Sulfonic Acid manufactured under the name "MEXORYL SL" by CHIMEX,
Camphor Benzalkonium Methosulfate manufactured under the name "MEXORYL SO" by CHIMEX,

The preferred hydrophilic UVB filter is Phenylbenzimidazole Sulfonic Acid.

### V/ Combined lipophilic UVA and UVB filters

### Benzophenone derivatives

Benzophenone-1 particularly sold under the trade name "UVINUL 400" by BASF;
Benzophenone-2 particularly sold under the trade name "UVINUL D50" by BASF;
Benzophenone-3 or Oxybenzone particularly sold under the trade name "UVINUL M40" by BASF;
Benzophenone-6 particularly sold under the trade name "Helisorb 11" by Norquay;
Benzophenone-8 particularly sold under the trade name "Spectra-Sorb UV-24" by American Cyanamid;
Benzophenone-10;
Benzophenone-11;
Benzophenone-12;

### Phenyl benzotriazole derivatives:

Drometrizole Trisiloxane particularly sold under the name "Silatrizole" by RHODIA CHIMIE or manufactured under the name "Meroxyl XL" by CHIMEX;
Methylene bis-Benzotriazolyl Tetramethylbutylphenol, sold in solid form particularly under the trade name "MIXXIM BB/100" by FAIRMOUNT CHEMICAL or in micronized form in aqueous dispersion particularly under the trade name "TINOSORB M" by CIBA SPECIALTY CHEMICALS;

### Benzoxazole derivatives:

2,4-bis-[5-1(dimethylpropyl)benzoxazol-2-yl-(4-phenyl)-imino]-6-(2-ethylhexyl)-imino-1,3,5-triazine particularly sold under the name Uvasorb K2A by Sigma 3V.

The preferential lipophilic organic UVA and UVB filters are selected from:
Drometrizole Trisiloxane;
Methylene bis-Benzotriazolyl Tetramethylbutylphenol;
Bis-Ethylhexyloxyphenol Methoxyphenyl Triazine.

### VI/ Combined hydrophilic UVA and UVB filters

Benzophenone derivatives comprising at least one sulfonic radical such as Benzophenone-4 particularly sold under the trade name "UVINUL MS 40" by BASF, Benzophenone-5 and
Benzophenone-9.

The composition according to the invention may also comprise mineral filters which are pigments. The pigments may be coated or uncoated.

The coated pigments are pigments which have undergone one or a plurality of chemical, electronic, mechanochemical and/or mechanical surface treatments with compounds as described for example in Cosmetics & Toiletries, February 1990, Vol. 105, p. 53-64, such as amino acids, beeswax, fatty acids, fatty alcohols, anionic surfactants, lecithins, sodium, potassium, zinc, iron or aluminum salts of fatty acids, metal (titanium or aluminum) alkoxides, polyethylene, silicones, proteins (collagen, elastin), alkanolamines, silicon oxides, metal oxides or sodium hexametaphosphate.

According to the prior art, the silicones are organosilicate polymers or oligomers with a linear or cyclic, branched or cross-linked structure, with a variable molecular weight, obtained by polymerization and/or polycondensation of suitably functionalized silanes, and essentially consisting of a repetition of primary structural units wherein the silicon atoms are interconnected by oxygen atoms (siloxane bond), optionally substituted hydrocarbon radicals being directly bound via a carbon atom on said silicon atoms.

The term "silicones" also covers the silanes required for the preparation thereof, particularly, alkyl silanes.

The silicones used for coating pigments suitable for the present invention are preferably selected from the group containing alkyl silanes, polydialkylsiloxanes, and polyalkylhydrogen siloxanes. More preferentially, the silicones are selected from the group containing octyl trimethyl silane, polydimethylsiloxanes and polymethylhydrogen siloxanes.

Obviously, prior to the treatment thereof with silicone, the metal oxide pigments may have been treated with other surface agents, particularly cerium oxide, alumina, silica, aluminum compounds, silicon compounds, or mixtures thereof.

The coated pigments are, for example, titanium oxides coated with:
- silica such as the product "SUNVEIL" from IKEDA and the product "Eusolex T-AVO" from MERCK
- silica and iron oxide such as the product "SUNVEIL F" from IKEDA,
- silica and alumina such as the products "MICROTITANIUM DIOXIDE MT 500 SA" and "MICROTITANIUM DIOXIDE MT 100 SA" from TAYCA, "TIOVEIL" from TIOXIDE, and "Mirasun TiW 60" from Rhodia,
- alumina such as the products "TIPAQUE TTO-55 (B)" and "TIPAQUE TTO-55 (A)" from ISHIHARA, and "UVT 14/4" from KEMIRA,
- alumina and aluminum stearate such as the product "MICROTITANIUM DIOXIDE MT 100 TV, MT 100 TX, MT 100 Z, MT-01" from TAYCA, the products "Solaveil CT-10 W", "Solaveil CT 100" and "Solaveil CT 200" from UNIQEMA,
- silica, alumina and alginic acid such as the product "MT-100 AQ" from TAYCA,
- alumina and aluminum laurate such as the product "MICROTITANIUM DIOXIDE MT 100 S" from TAYCA,
- iron oxide and iron stearate such as the product "MICROTITANIUM DIOXIDE MT 100 F" from TAYCA,
- zinc oxide and zinc stearate such as the product "BR351" from TAYCA,
- silica and alumina and treated with a silicone such as the products "MICROTITANIUM DIOXIDE MT 600 SAS", "MICROTITANIUM DIOXIDE MT 500 SAS" or "MICROTITANIUM DIOXIDE MT 100 SAS" from TAYCA,
- silica, alumina, aluminum stearate and treated with a silicone such as the product "STT-30-DS" from TITAN KOGYO,
- silica and treated with a silicone such as the product "UV-TITAN X 195" from KEMIRA, or the product SMT-100 WRS from TAYCA.
- alumina and treated with a silicone such as the products "TIPAQUE TTO-55 (S)" from ISHIHARA, or "UV TITAN M 262" from KEMIRA,
triethanolamine such as the product "STT-65-S" from TITAN KOGYO,
- stearic acid such as the product "TIPAQUE TTO-55 (C)" from ISHIHARA,
- sodium hexametaphosphate such as the product "MICROTITANIUM DIOXIDE MT 150 W" from TAYCA.

Further titanium oxide pigments treated with a silicone are for example TiO₂ treated with octyl trimethyl silane such as that sold under the trade name "T 805" by DEGUSSA SILICES, TiO₂ treated with a polydimethylsiloxane such as that sold under the trade name "70250 Cardre UF TiO2Sl3" by CARDRE, anatase/rutile TiO₂ treated with a polydimethylhydrogen siloxane such as that sold under the trade name "MICRO TITANIUM DIOXYDE USP GRADE HYDROPHOBIC" by COLOR TECHNIQUES.

The uncoated titanium oxide pigments are for example sold by TAYCA under the trade names "MICROTITANIUM DIOXIDE MT 500 B" or "MICROTITANIUM DIOXIDE MT600 B", by DEGUSSA under the name "P 25", by WACKHER under the name "Oxyde de titane transparent PW", by MIYOSHI KASEI under the name "UFTR", by TOMEN under the name "ITS" and by TIOXIDE under the name "TIOVEIL AQ".

The uncoated zinc oxide pigments are for example
- those marketed under the name "Z-cote" by Sunsmart;
- those marketed under the name "Nanox" by Elementis;
- those marketed under the name "Nanogard WCD 2025" by Nanophase Technologies;

The coated zinc oxide pigments are for example
- those marketed under the name "Z-COTE HP1" by SUNSMART (ZnO coated with dimethicone);
- those marketed under the name "CS-5 zinc oxide" by Toshibi (ZnO coated with polymethylhydrogen siloxane);
- those marketed under the name "Nanogard Zinc Oxide FN" by Nanophase Technologies (in 40% dispersion in Finsolv TN, C12-C15 alcohol benzoate);
- those marketed under the name "DAITOPERSION ZN-30" and "DAITOPERSION Zn-50" by Daito (dispersions in cyclopolymethylsiloxane/oxyethylenated polydimethylsiloxane, containing 30% or 50% zinc oxides coated with silica and polymethylhydrogen siloxane);
- those marketed under the name "NFD Ultrafine ZnO" by Daikin (ZnO coated perfluoroalkyl phosphate and perfluoroalkylethyl-based copolymer in dispersion in cyclopentasiloxane);
- those marketed under the name "SPD-Z1" by Shin-Etsu (ZnO coated with silicone-grafted acrylic polymer, dispersed in cyclodimethylsiloxane);
- those marketed under the name "Escalol Z100" by ISP (ZnO treated with alumina and dispersed in the ethylhexyl methoxycinnamate / PVP-hexadecene copolymer / methicone mixture);
- those marketed under the name "Fuji ZnO-SMS-10" by Fuji Pigment (ZnO coated with silica and polymethylsilsesquioxane);
- those marketed under the name "Nanox Gel TN" by Elementis (ZnO in 55% dispersion in C12-C15 alcohol benzoate with hydroxystearic acid polycondensate).

The uncoated cerium oxide pigments are sold for example under the name "COLLOIDAL CERIUM OXIDE" by RHONE POULENC.

The uncoated iron oxide pigments are for example sold by ARNAUD under the names "NANOGARD WCD 2002 (FE 45B)", "NANOGARD IRON FE 45 BL AQ", "NANOGARD FE 45R AQ, "NANOGARD WCD 2006 (FE 45R)", or by MITSUBISHI under the name "TY-220".

The coated iron oxide pigments are for example sold by ARNAUD under the names "NANOGARD WCD 2008 (FE 45B FN)", "NANOGARD WCD 2009 (FE 45B 556)", "NANOGARD FE 45 BL 345", "NANOGARD FE 45 BL", or by BASF under the name "TRANSPARENT IRON OXIDE".

It is also possible to cite metal oxide mixtures, particularly of titanium dioxide and cerium dioxide, including the mixture of equal weights of titanium dioxide and cerium dioxide coated with silica, sold by IKEDA under the name "SUNVEIL A", and the mixture of titanium dioxide and zinc dioxide coated with alumina, silica and silicone such as the product "M 261" sold by KEMIRA or coated with alumina, silica and glycerin such as the product "M 211" sold by KEMIRA.

The pigments may be introduced into the compositions according to the invention as is or in pigment paste form, i.e. in a mixture with a dispersion agent, as described for example in the document GB-A-2206339.

The additional UV filters are generally present in the compositions according to the invention in proportions ranging from 0.01 to 20% by weight with respect to the total weight of the composition, and preferably ranging from 0.1 to 10% by weight with respect to the total weight of the composition.

The composition may further comprise at least one additional ingredient intended to provide an immediate visual effect. This particularly includes fillers with a soft-focus effect or agents promoting naturally pink skin color.

Agents promoting naturally pink skin color include, for example, self-tanning agents, i.e. an agent which, applied on the skin, particularly on the face, makes it possible to obtain a tanning effect more or less similar to that liable to result from prolonged exposure to the sun (natural tan) or a UV lamp;

Examples of self-tanning agents particularly include:
dihydoxyacetone (DHA),
erythrulose, and
the association of a catalytic system formed from:
   manganese and/or zinc salts and oxides, and
   alkaline and/or alkaline earth hydrogen carbonates.

The self-tanning agents are generally selected from mono or polycarbonyl compounds such as for example isatin, alloxan, ninhydrin, glyceraldehyde, mesotartaric aldehyde, glutaraldehyde, erythrulose, pyrazolin-4,5-dione derivatives as described in the patent application FR 2 466 492 and WO 97/35842, dihydroxyacetone (DHA), 4,4-dihydroxypyrazolin-5-one derivatives as described in the patent application EP 903 342. DHA is preferably used.

DHA may be used in free and/or encapsulated form for example in lipid vesicles such as liposomes, particularly described in the application WO 97/25970.

As a general rule, the self-tanning agent, particularly DHA, is present in an amount ranging from 0.01 to 20 % by weight, and preferably in an amount between 0.1 and 10% of the total weight of the composition.

Further colorants suitable for modifying the color produced by the self-tanning agent may be used.

These colorants may be selected from synthetic or natural direct colorants.

These colorants may be selected for example from red or orange fluorane type colorants such as those described in the patent application FR2840806. For example, this includes the following colorants:
- tetrabromofluoroscein or eosin known as the CTFA name: Cl 45380 or Red 21
- phloxine B known as the CTFA name: Cl 45410 or Red 27
- diiodofluorescein known as the CTFA name Cl 45425 or Orange 10;
- dibromofluorescein known as the CTFA name: Cl 45370 or Orange 5.
- tetrabromofluoroscein sodium salt known as the CTFA name: Cl 45380 (Na salt) or Red 22
- phloxine B sodium salt known as the CTFA name: Cl 45410 (Na salt) or Red 28
- diiodofluorescein sodium salt known as the CTFA name: Cl 45425 (Na salt) or Orange 11;
- erythrosine known as the CTFA name: Cl 45430 or Acid Red 51.
- phloxine known as the CTFA name: Cl 45405 or Acid Red 98.

These colorants may also be selected from anthraquinones, caramel, carmine, carbon black, azulene blues, methoxalene, trioxalene, guajazulene, chamuzulene, rose bengal, cosine 10B, cyanosin, daphinin.

These colorants may also be selected from indole derivatives such as monohydroxyindoles as described in the patent FR2651126 (i.e.: 4-, 5-, 6- or 7-hydroxyindole) or di-hydroxyindoles as described in the patent EP-B-0425324 (i.e.: 5,6-dihydroxyindole, 2-methyl 5,6-dihydroxyindole, 3-methyl 5,6-dihydroxyindole, 2,3-dimethyl 5,6-dihydroxyindole).
The compositions according to the present invention may further comprise conventional cosmetic adjuvants particularly selected from fatty substances, particularly oils, waxes, organic solvents, ionic or non-ionic thickeners, hydrophilic or lipophilic, softeners, moistening agents, opacifiers, stabilizers, emollients, silicones, anti-foaming agents, perfumes, preservatives, anionic, cationic, non-ionic, zwitterionic or amphoteric surfactants, active substances, fillers, polymers, propellants, alkalinizing or acidifying agents or any other ingredient routinely used in the cosmetic and/or dermatological field.

The fatty substances may consist of an oil or a wax or mixtures thereof. The term oil refers to a liquid compound at ambient temperature. The term wax refers to a solid or substantially solid compound at ambient temperature, wherein the melting point is generally greater than 35.

The oils include, for example mineral oils (paraffin oil); vegetable oils (sweet almond, macadamia, blackcurrant seed, jojoba); synthetic oils such as perhydrosqualene, fatty alcohols, fatty amides (such as isopropyl lauroyl sarcosinate sold under the name "Eldew SL-205" by Ajinomoto), fatty acids or esters such as C12-C15 alcohol benzoate sold under the trade name "Finsolv TN" or "Witconol TN" by WITCO, 2-ethylphenyl benzoate such as the commercial product sold under the name X-TEND 226 ® by ISP, octyl palmitate, isopropyl lanolate, triglycerides including those of capric/caprylic acids, dicaprylyl carbonate sold under the name "Cetiol CC" by Cognis), oxyethylene or oxypropylene fatty esters and ethers; silicone oils (cyclomethicone, polydimethysiloxanes or PDMS) or fluorinated oils, polyalkylenes, trialkyl trimellitates such as tridecyl trimellitate.

The waxes include, for example, carnauba wax, beeswax, hydrogenated castor oil, polyethylene waxes and polymethylene waxes such as that sold under the name Cirebelle 303 by SASOL.

The organic solvents include, for example lower alcohols and polyols. These may be selected from glycols and glycol ethers such as ethylene glycol, propylene glycol, butylene glycol, dipropylene glycol or diethylene glycol.

The hydrophilic thickeners include, for example, carboxyvinyl polymers such as Carbopols (Carbomers) and Pemulens (acrylate/C10-C30-alkylacrylate copolymer); polyacrylamides such as for example cross-linked copolymers sold under the names Sepigel 305 (C.T.F.A. name: polyacrylamide/C13-14 isoparaffin/Laureth 7) or Simulgel 600 (C.T.F.A. name: acrylamide / sodium acryloyldimethyltaurate copolymer / isohexadecane / polysorbate 80) by Seppic; 2-acrylamido 2-methylpropane sulfonic acid polymers and copolymers, optionally cross-linked and/or neutralized, such as poly(2-acrylamido 2-methylpropane sulfonic acid) marketed by Hoechst under the trade name "Hostacerin AMPS" (CTFA name: ammonium polyacryloyldimethyl taurate or SIMULGEL 800 marketed by SEPPIC (CTFA name: sodium polyacryolyldimethyl taurate / polysorbate 80 / sorbitan oleate); 2-acrylamido 2-methylpropane sulfonic acid and hydroxyethyl acrylate copolymers such as SIMULGEL NS and SEPINOV EMT 10 marketed by SEPPIC; cellulose derivatives such as hydroxyethylcellulose; polysaccharides and particularly gums such as Xanthan gum; hydrophilic or hydro-dispersible silicone derivatives such as acrylic silicones, silicone polyethers and cationic silicones and mixtures thereof.

The lipophilic thickeners include, for example, synthetic polymers such as poly C10-C30 alkyl acrylates sold under the name "INTELIMER IPA 13-1" and "INTELIMER IPA 13-6" by Landec or modified clays such as hectorite and derivatives thereof, such as the products marketed under the names of Bentone.

Obviously, those skilled in the art would take care to select the optional complementary compound(s) cited above and/or the amounts thereof such that the advantageous properties intrinsically associated with the compositions according to the invention are not, or substantially not, impaired by the envisaged addition(s), particularly the enhancement in the protection of the skin and/or hair and/or lips against sun rays.

The compositions according to the invention may be prepared according to techniques well-known to those skilled in the art. They may particularly be in the form of a simple or complex emulsion (O/W, W/O, O/W/O or W/O/W) such as a cream, milk or cream gel; in the form of an aqueous gel; in the form of a lotion. They may optionally be packaged in an aerosol and be presented in the form of a foam or spray.

The compositions according to the invention may be presented in the form of an oil-in-water or water-in-oil emulsion.

The emulsification methods suitable for use are of the blade or impeller, rotor-stator and HHP type.

It is also possible, by means of HHP (between 50 and 800 bar), to obtain stable dispersions with droplet sizes as small as 100 nm.

The emulsions generally contain at least one emulsifying surfactant selected from amphoteric, anionic, cationic or non-ionic emulsifying surfactants, used alone or in a mixture. The emulsifiers are suitably selected according to the emulsion to be obtained (W/O or O/W).

The emulsifying surfactants suitable for use for preparing W/O emulsions include, for example, alkyl esters or sorbitan, glycerol or sugar ethers; silicone surfacants such as dimethicone copolyols such as the mixture of cyclomethicone and dimethicone copolyol, sold under the name "DC 5225 C" by Dow Corning, and alkyl-dimethicone copolyols such as Laurylmethicone copolyol sold under the name "Dow Corning 5200 Formulation Aid" by Dow Corning; Cetyl dimethicone copolyol such as the product sold under the name Abil EM 90R by Goldschmidt and the mixture of cetyl dimethicone copolyol, polyglycerol isostearate (4 moles) and hexyl laurate under the name ABIL WE 09 by Goldschmidt. It is also possible to add one or a plurality of co-emulsifiers, which, advantageously, may be selected from the group comprising polyol alkyl esters.

The polyol alkyl esters particularly include polyethyleneglycol esters such as PEG-30 Dipolyhydroxystearate such as the product marketed under the name Arlacel P135 by ICI.

The glycerol and/or sorbitan esters include, for example, polyglycerol isostearate, such as the product marketed under the name Isolan Gl 34 by Goldschmidt; sorbitan isostearate, such as the product marketed under the name Arlacel 987 by ICI; sorbitan isostearate and glycerol, such as the product marketed under the name Arlacel 986 by ICI, and mixtures thereof.

For the O/W emulsions, the emulsifying surfactants include, for example, non-ionic emulsifiers such as oxyalkylene (more specifically polyoxyethene) fatty acid and glycerol esters; oxyalkylene fatty acid and sorbitan esters; oxyalkylene (oxyethylene and/or oxypropylene) fatty acid esters such as the PEG-100 Stearate/Glyceryl Stearate mixture marketed for example by ICI under the name Arlacel 165; oxyalkylene (oxyethylene and/or oxypropylene) fatty alcohol ethers; sugar esters such as sucrose stearate; fatty alcohol and sugar ethers, particularly alkylpolyglucosides (APG) such as decylglucoside and laurylglucoside marketed for example by Henkel under the respective names Plantaren 2000 and Plantaren 1200, cetostearylglucoside optionally in a mixture with cetostearyl alcohol, marketed for example under the name Montanov 68 by Seppic, under the name Tegocare CG90 by Goldschmidt and under the name Emulgade KE3302 by Henkel, and arachidyl glucoside, for example in the form of the mixture of arachidic and behenic alcohols and arachidylglucoside marketed under the name Montanov 202 by Seppic. According to one particular embodiment of the invention, the alkylpolyglucoside mixture as defined above with the corresponding fatty alcohol may be in the form of a self-emulsifying composition, as described for example in the document WO-A-92/06778.

In the case of an emulsion, the aqueous phase thereof may comprise a non-ionic vesicular dispersion prepared according to known methods (Bangham, Standish and Watkins. J. Mol. Biol. 13, 238 (1965), FR 2 315 991 and FR 2 416 008).

The compositions according to the invention may be applied in a large number of treatments, particularly cosmetic treatments, of the skin, lips and/or hair, including the scalp, particularly for protecting and/or treating the skin, lips and/or hair, and/or for makeup for the skin and/or lips.

The cosmetic compositions according to the invention may for example by used as a makeup product.

The cosmetic compositions according to the invention may for example be used in a method of treatment and/or sun protection product for the face and/or body with a liquid to semiliquid consistency, such as milks, more or less unctuous creams, cream gels, pastes. They may optionally be packaged in an aerosol and be presented in the form of a foam or spray.

The compositions according to the invention in the form of fluid lotions suitable for vaporization according to the invention are applied on the skin or hair in the form of fine particles by means of pressurization devices. The devices suitable for the invention are well known to those skilled in the art and comprise non-aerosol pumps or "atomizers", aerosol containers comprising a propellant and aerosol pumps using compressed air as a propellant. The latter are described in the patents US 4,077,441 and US 4,850,517 (an integral part of the content of the description).

The compositions packaged in an aerosol suitable for the invention generally contain conventional propellants such as for example the hydrofluorinated compounds dichlorodifluoromethane, difluoroethane, dimethylether, isobutane, n-butane, propane, trichlorofluoromethane. They are preferably present in amounts ranging from 15 to 50% by weight with respect to the total weight of the composition.

The compositions packaged in an aerosol suitable for the invention generally contain conventional propellants such as for example the hydrofluorinated compounds dichlorodifluoromethane, difluoroethane, dimethylether, isobutane, n-butane, propane, trichlorofluoromethane. They are preferably present in amounts ranging from 15 to 50% by weight with respect to the total weight of the composition.

### In the figures:

- Figure 1 represents the absorption spectrum of the reference filter tested with, on the x-axis, the wavelength in nanometers and, on the y-axis, the absorbance (Abs);
- Figure 2 represents a histogram of the fluorescence (%) as a function of the compositions tested.

The concrete, but in no way limiting, examples illustrating the invention will now be given.

Furthermore, in the examples, all the percentages are given by weight, unless specified otherwise, and the temperature is expressed in degrees Celsius unless specified otherwise, and the pressure is atmospheric pressure, unless specified otherwise. In the examples, the amounts of the ingredients of the compositions are given as a % by weight with respect to the total weight of the composition.

### Examples

Example 1: Study of skin protection under the effect of UVA by a composition according to the invention

The purpose of this study was to express the protection performance with respect to UVA stress. Baicalin is evaluated alone and according to the invention, in association with a sunscreen composition 1 comprising a system filtering UVA comprising at least one bis-resorcinyl triazine derivative.

### Sunscreen composition 1

| **Phase** | **Chemical name** | **US INCI name** | **Concentration** |
|---|---|---|---|
| | GLYCERYL MONO/DISTEARATE / POLYETHYLENE GLYCOL STEARATE MIXTURE (100 OE) | GLYCERYL STEARATE (and) PEG-100 STEARATE | 1 |
| | POLY DIMETHYLSILOXANE (VISCOSITY: 350 CST) | DIMETHICONE | 0,5 |
| | POLYMETHYLENE WAX HAVING A MELTING POINT OF APPROXIMATELY 64°C | SYNTHETIC WAX | 1 |
| | OCTANE-1,2 DIOL | CAPRYLYL GLYCOL | 0,4 |
| | ISOPROPYL N-LAUROYLSARCOSINATE | ISOPROPYL LAUROYL SARCOSINATE | 10 |
| | TRIAZINE DERIVATIVE (BEMT) = (2,4-BIS((4-(2-ETHYL-HEXYLOXY)-2-HYDROXY)-PHENYL)-6-(4-METHOXYPHENYL)-(1,3,5)-TRIAZINE) | BIS-ETHYLHEXYLOXYPHENOL METHOXYPHENYL TRIAZINE | 1 |
| | 2-ETHYL HEXYL 2-CYANO-3,3-DIPHENYLACRYLATE | OCTOCRYLENE | 5 |
| | 4-TERTI OB UTYL-4'-M ETHOXY-DIBENZOYLMETHANE | BUTYL METHOXYDIBENZOYLMETHANE | 1,5 |
| | TRIETHANOLAMINE | TRIETHANOLAMINE | 0,2 |
| | PHENOXY-2 ETHANOL | PHENOXYETHANOL | 0,7 |
| | MICROBIOLOGICALLY CLEAN DEIONIZED WATER | WATER | 53,63 |
| | ETHYLENE DIAMINE TETRACETIC ACID, DISODIUM SALT, 2 H2O | DISODIUM EDTA | 0,1 |
| | GLYCEROL | GLYCERIN | 6 |
| | PROPYLENE GLYCOL | PROPYLENE GLYCOL | 6 |
| | ACRYLIC ACID/STEARYL METHACRYLATE COPOLYMER POLYMERIZED IN AN ETHYL ACETATE/CYCLOHEXANE MIXTURE | ACRYLATES/C10-30 ALKYL ACRYLATE CROSSPOLYMER | 0,25 |
| | XANTHAN: POLYSACCHARIDES: GLUCOSE/MANNOSE/GLUCURONIC ACID (40/30/30) | XANTHAN GUM | 0,2 |
| | DEIONIZED WATER | WATER | 1 |
| A1 | PLANT FATTY ACIDS (MAINLY STEARIC ACID) | STEARIC ACID | 1 |
| A2 | CYCLOHEXA DIMETHYLSILOXANE (VISCOSITY: 8 CST) | CYCLOHEXASILOXANE | 3 |
| B1 | B-B'CAMPHOSULFONIC ACID [1-4 DIVINYLBENZENE] IN AQUEOUS SOLUTION | TEREPHTHALYLIDENE DICAMPHOR SULFONIC ACID | 0,3 |
| B1 | TRIETHANOLAMINE | TRIETHANOLAMINE | 0,05 |
| B2 | NON-STABILIZED MONOPOTASSIUM MONO-CETYL PHOSPHATE | POTASSIUM CETYL PHOSPHATE | 1 |
| C | ISO-HEXADECANE | ISOHEXADECANE | 3 |
| D | TRIETHANOLAMINE | TRIETHANOLAMINE | 0,17 |
| E | DENATURED ABSOLUTE ETHYL ALCOHOL | ALCOHOL DENAT. | 3 |

### Placebo composition 2

| Phase | Chemical name | US INCI name | Concentration |
|---|---|---|---|
| | GLYCERYL MONO/DISTEARATE / POLYETHYLENE GLYCOL STEARATE MIXTURE (100 OE) | GLYCERYL STEARATE (and) PEG-100 STEARATE | 1 |
| | POLY DIMETHYLSILOXANE (VISCOSITY: 350 CST) | DIMETHICONE | 0,5 |
| | POLYMETHYLENE WAX HAVING A MELTING POINT OF APPROXIMATELY 64°C | SYNTHETIC WAX | 1 |
| | OCTANE-1,2 DIOL | CAPRYLYL GLYCOL | 0,4 |
| | ISOPROPYL N-LAUROYLSARCOSINATE | ISOPROPYL LAUROYL SARCOSINATE | 18,25 |
| | TRIETHANOLAMINE | TRIETHANOLAMINE | 0,2 |
| | PHENOXY-2 ETHANOL | PHENOXYETHANOL | 0,7 |
| | MICROBIOLOGICALLY CLEAN DEIONIZED WATER | WATER | 53,18 |
| | ETHYLENE DIAMINE TETRACETIC ACID, DISODIUM SALT, 2 H2O | DISODIUM EDTA | 0,1 |
| | GLYCEROL | GLYCERIN | 6 |
| | PROPYLENE GLYCOL | PROPYLENE GLYCOL | 6 |
| | ACRYLIC ACID/STEARYL METHACRYLATE COPOLYMER POLYMERIZED IN AN ETHYL ACETATE/CYCLOHEXANE MIXTURE | ACRYLATES/C10-30 ALKYL | |
| | | ACRYLATE CROSSPOLYMER | 0,25 |
| | XANTHAN: POLYSACCHARIDES: GLUCOSE/MANNOSE/GLUCURONIC ACID (40/30/30) | XANTHAN GUM | 0,2 |
| | DEIONIZED WATER | WATER | 1 |
| A1 | PLANT FATTY ACIDS (MAINLY STEARIC ACID) | STEARIC ACID | 1 |
| A2 | CYCLOHEXA DIMETHYLSILOXANE (VISCOSITY: 8 CST) | CYCLOHEXASILOXANE | 3 |
| B1 | TRIETHANOLAMINE | TRIETHANOLAMINE | 0,05 |
| B2 | NON-STABILIZED MONOPOTASSIUM MONO-CETYL PHOSPHATE | POTASSIUM CETYL PHOSPHATE | 1 |
| C | ISO-HEXADECANE | ISOHEXADECANE | 3 |
| D | TRIETHANOLAMINE | TRIETHANOLAMINE | 0,17 |
| E | DENATURED ABSOLUTE ETHYL ALCOHOL | ALCOHOL DENAT. | 3 |

### 1.1. Method and protocols

Under our experimental conditions, exposure of NHF (Normal Human Fibroblasts) to UVA induces the generation of activated oxygen species, measured using the DCFH-DA test.

The culture medium as follows:
- 500 ml of DMEM base medium with Glutamax 1,
- 50 ml of FCS,
- 5 ml of 250µg/ml Fungizone Ampb B,
- 5 ml 200 mM L-glutamine
- 5 ml pennicilin-streptomycin

### 1.1.1. Conditions for evaluating protection against UVA radiation

The NHF cells are pretreated with the active substances for 24 hours before exposure to UV. During the exposure of the fibroblasts to UVA, the reference formula ("Reference filter" consisting of sunscreen composition 1) and the placebo formula (placebo composition 2) are applied on a rough PMMA plate in the form of homogeneous and regular deposition at a rate of 0.6mg/cm². The plate is then positioned over the culture plate. The active substances (baicalin and/or system filtering UVA comprising at least one bis-resorcinyl triazine derivative, referred to as the reference filter) under evaluation are present during the solar simulation. The following combinations are evaluated:
- NHF + UVA + Placebo;
- NHF + UVA + Placebo + baicalin;
- NHF + UVA + Reference filter;
- NHF + UVA + Reference filter + baicalin.

For the NHF cells treated with the active substance, a control plate is kept protected from light.

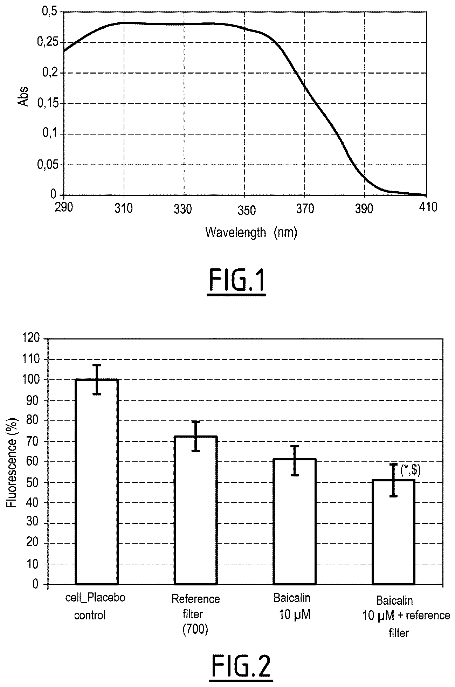

The absorption spectrum of the reference filter (sunscreen composition 1) tested is shown in figure 1. This filter comprises a bis-resorcinyl triazine derivative: TINOSORB S; and a dibenzoylmethane derivative: Avobenzone; and a compound filtering UVA derived from benzylidene camphor: Terephthalylidene Dicamphor Sulfonic Acid, associated with an acrylate: octocrylene. This reference filter is already an optimal filter according to the relevant prior art, per se, considered to be technically difficult to improve in respect to the ability to protect the skin against UV.

### 1.1.2. Exposure conditions

The UVA are output with the Oriel solar simulator (Xenon lamp, 1000Watts), equipped with a WG360/2mm filter. To obtain the entire UVA range, the UG11 visible section is not used (however, it should be noted that the dichroic mirror of the solar simulator filters a large portion of the visible range (85%)). The UVA emitted can be referred to as long UVA as it is within the wavelengths of 340 to 400 nm.

### 1.1.3. DCFHDA test: Evaluation of UVA-induced oxidative stress

The protection against UVA stress is evaluated by means of a DCFH-DA test as a marker of overall intracellular oxidative stress. This makes it possible to directly evaluate the negative impact of UVA on a cellular level, and particularly on harmful oxidative stress for the skin, hair and lips, particularly on skin tissue elasticity, promoting the appearance of wrinkles giving rise to premature skin ageing.

The use of DCFH-DA as a marker of overall intracellular oxidative stress is based on the physicochemical properties thereof. It consists of an apolar and non-ionic molecule capable of diffusion via cell membranes. Once inside the cell, the DCFH-DA is hydrolyzed by intracellular esterases to a non-fluorescent compound: DCFH or 2,7-dichlorofluorescin. In the presence of activated oxygen species (H₂O₂; OH°), DCFH is rapidly oxidized to a highly fluorescent compound: DCF or 2,7-dichlorofluorescein.

The fibroblasts, optionally in the presence of 10µM Baicalin for 24 hours, are rinsed with PBS⁺ and incubated in the presence of DCFH-DA [20µM] for 30 minutes at 37°C and protected from light. After removing the probes and 2 rinses, the cells are again placed in contact with the active substances (10µM) in PBS+. PMMA plates whereon either Vaseline (filter-free control) or the reference filtering formula have been applied are deposited on top of the cells. Baicalin is introduced into the culture medium at a concentration of 10 micromoles. They are then exposed to 20J/cm² of UVA (WG360 filter). The fluorescence of DCF is evaluation immediately after exposure to UVA, by spectrofluorimetry (Excitation 480nm; Emission 530nm).

### 1.2/ RESULTS

The evaluation of 10µM Baicalin in the DCFH-DA test demonstrated the following results (figure 2):
- sunscreen composition 1 partly protects fibroblasts against UVA-induced ROS (Reactive Oxygen Species): 23-27% protection;
- associating sunscreen composition 1 with baicalin significantly improves the level of biological protection provided to cells in the UVA range, with a 50% efficacy for the "Baicalin + Reference filter" association.

In this way, these results demonstrate the surprisingly enhanced efficacy due to the association of baicalin and a filtering system comprising a UVA filter comprising at least one bis-resorcinyl triazine derivative, for protecting the skin and/or lips and/or hair against solar radiation, particularly UV.

These results provide a means other than that consisting of using large amounts of UV filters, particularly UVA filters, for combating premature ageing of the skin and/or lips and/or hair effectively under conditions of use and in concentrations acceptable for those skilled in the art.

Finally, these results particularly provide compositions, particularly cosmetic compositions, for preventing and/or combating premature ageing of the skin and/or lips due to UVA radiation and for protecting the skin and/or lips and/or hair against this form of radiation and free radicals.

### 21 EXAMPLES OF FORMULATIONS

### 2.1 FORMULATION A

| **CHEMICAL NAME** | **EU INCI NAME** | **CONCENTRATION** |
|---|---|---|
| MICROBIOLOGICALLY CLEAN DEIONIZED WATER | AQUA | 64,23 |
| GLYCEROL | GLYCERIN | 6 |
| PROPYLENE GLYCOL | PROPYLENE GLYCOL | 6 |
| ISOPROPYL N-LAUROYLSARCOSINATE | ISOPROPYL LAUROYL SARCOSI NATE | 5 |
| DENATURED ABSOLUTE ETHYL ALCOHOL | ALCOHOL DENAT. | 3 |
| ISO-HEXADECANE | ISOHEXADECANE | 3 |
| CYCLOHEXA DIMETHYLSILOXANE (VISCOSITY: 8 CST) | CYCLOHEXASILOXANE | 3 |
| TRIAZINE DERIVATIVE (BEMT) = (2,4-BIS((4-(2-ETHYL-HEXYLOXY)-2-HYDROXY)-PHENYL)-6-(4-METHOXYPHENYL)-(1,3,5)-TRIAZINE) | BIS-ETHYLHEXYLOXYPHENOL METHOXYPHENYL TRIAZINE | 1 |
| NON-STABILIZED MONOPOTASSIUM MONO-CETYL PHOSPHATE | POTASSIUM CETYL PHOSPHATE | 1 |
| PLANT FATTY ACIDS (MAINLY STEARIC ACID) | STEARIC ACID | 1 |
| GLYCERYL MONO/DISTEARATE / POLYETHYLENE GLYCOL STEARATE MIXTURE (100 OE) | GLYCERYL STEARATE (AND) PEG-100 STEARATE | 1 |
| POLYMETHYLENE WAX HAVING A MELTING POINT OF APPROXIMATELY 64°C | SYNTHETIC WAX | 1 |
| DIISOPROPYL SEBACATE | DIISOPROPYL SEBACATE | 1 |
| DEIONIZED WATER | AQUA | 1 |
| PHENOXY-2 ETHANOL | PHENOXYETHANOL | 0,7 |
| POLY DIMETHYLSILOXANE (VISCOSITY: 350 CST) | DIMETHICONE | 0,5 |
| OCTANE-1,2 DIOL | CAPRYLYL GLYCOL | 0,4 |
| ACRYLIC ACID/STEARYL METHACRYLATE COPOLYMER POLYMERIZED IN AN ETHYL ACETATE/CYCLOHEXANE MIXTURE | ACRYLATES/C10-30 ALKYL ACRYLATE CROSSPOLYMER | 0,25 |
| BAICALIN (PURITY >95%) SCUTELLARIA BAICALENSIS ROOT EXTRACT | SCUTELLARIA BAICALENSIS EXTRACT | 0,2 |
| TRIETHANOLAMINE | TRIETHANOLAMINE | 0,2 |
| XANTHAN: POLYSACCHARIDES: GLUCOSE/MANNOSE/GLUCURONIC ACID (40/30/30) | XANTHAN GUM | 0,2 |
| TRIETHANOLAMINE | TRIETHANOLAMINE | 0,17 |
| ETHYLENE DIAMINE TETRACETIC ACID, DISODIUM SALT, 2 H2O | DISODIUM EDTA | 0,1 |
| TRIETHANOLAMINE | TRIETHANOLAMINE | 0,05 |

### 2.1 FORMULATION B

| **CHEMICAL NAME** | **EU INCI NAME** | **CONCENTRATION** |
|---|---|---|
| MICROBIOLOGICALLY CLEAN DEIONIZED WATER | AQUA | 62,73 |
| GLYCEROL | GLYCERIN | 6 |
| PROPYLENE GLYCOL | PROPYLENE GLYCOL | 6 |
| ISOPROPYL N-LAUROYLSARCOSINATE | ISOPROPYL LAUROYL SARCOSINATE | 5 |
| DENATURED ABSOLUTE ETHYL ALCOHOL | ALCOHOL DENAT. | 3 |
| ISO-HEXADECANE | ISOHEXADECANE | 3 |
| CYCLOHEXA DIMETHYLSILOXANE (VISCOSITY: 8 CST) | CYCLOHEXASILOXANE | 3 |
| 4-TERTIOBUTYL-4'-METHOXY-DIBENZOYLMETHANE | BUTYL METHOXYDIBENZOYLMETHANE | 1,5 |
| TRIAZINE DERIVATIVE (BEMT) = (2,4-BIS((4-(2-ETHYL-HEXYLOXY)-2-HYDROXY)-PHENYL)-6-(4-METHOXYPHENYL)-(1,3,5)-TRIAZINE) | BIS-ETHYLHEXYLOXYPHENOL METHOXYPHENYL TRIAZINE | 1 |
| NON-STABILIZED MONOPOTASSIUM MONO-CETYL PHOSPHATE | POTASSIUM CETYL PHOSPHATE | 1 |
| PLANT FATTY ACIDS (MAINLY STEARIC ACID) | STEARIC ACID | 1 |
| GLYCERYL MONO/DISTEARATE / POLYETHYLENE GLYCOL STEARATE MIXTURE (100 OE) | GLYCERYL STEARATE (AND) PEG-100 STEARATE | 1 |
| POLYMETHYLENE WAX HAVING A MELTING POINT OF APPROXIMATELY 64°C | SYNTHETIC WAX | 1 |
| DIISOPROPYL SEBACATE | DIISOPROPYL SEBACATE | 1 |
| DEIONIZED WATER | AQUA | 1 |
| PHENOXY-2 ETHANOL | PHENOXYETHANOL | 0,7 |
| POLY DIMETHYLSILOXANE (VISCOSITY: 350 CST) | DIMETHICONE | 0,5 |
| OCTANE-1,2 DIOL | CAPRYLYL GLYCOL | 0,4 |
| ACRYLIC ACID/STEARYL METHACRYLATE COPOLYMER POLYMERIZED IN AN ETHYL ACETATE/CYCLOHEXANE MIXTURE | ACRYLATES/C10-30 ALKYL ACRYLATE CROSSPOLYMER | 0,25 |
| BAICALIN (PURITY >95%) SCUTELLARIA BAICALENSIS ROOT EXTRACT | SCUTELLARIA BAICALENSIS EXTRACT | 0,2 |
| TRIETHANOLAMINE | TRIETHANOLAMINE | 0,2 |
| XANTHAN: POLYSACCHARIDES: GLUCOSE/MANNOSE/GLUCURONIC ACID (40/30/30) | XANTHAN GUM | 0,2 |
| TRIETHANOLAMINE | TRIETHANOLAMINE | 0,17 |
| ETHYLENE DIAMINE TETRACETIC ACID, DISODIUM SALT, 2 H2O | DISODIUM EDTA | 0,1 |
| TRIETHANOLAMINE | TRIETHANOLAMINE | 0,05 |

### 2.1 FORMULATION C

| **CHEMICAL NAME** | **EU INCI NAME** | **CONCENTRATION** |
|---|---|---|
| MICROBIOLOGICALLY CLEAN DEIONIZED WATER | AQUA | 59,23 |
| GLYCEROL | GLYCERIN | 6 |
| PROPYLENE GLYCOL | PROPYLENE GLYCOL | 6 |
| ISOPROPYL N-LAUROYLSARCOSINATE | ISOPROPYL LAUROYL SARCOSINATE | 5 |
| 2-ETHYL HEXYL 2-CYANO-3,3-DIPHENYLACRYLATE | OCTOCRYLENE | 5 |
| DENATURED ABSOLUTE ETHYL ALCOHOL | ALCOHOL DENAT. | 3 |
| ISO-HEXADECANE | ISOHEXADECANE | 3 |
| CYCLOHEXA DIMETHYLSILOXANE (VISCOSITY: 8 CST) | CYCLOHEXASILOXANE | 3 |
| TRIAZINE DERIVATIVE (BEMT) = (2,4-BIS((4-(2-ETHYL-HEXYLOXY)-2-HYDROXY)-PHENYL)-6-(4-METHOXYPHENYL)-(1,3,5)-TRIAZINE) | BIS-ETHYLHEXYLOXYPHENOL METHOXYPHENYL TRIAZINE | 1 |
| NON-STABILIZED MONOPOTASSIUM MONO-CETYL PHOSPHATE | POTASSIUM CETYL PHOSPHATE | 1 |
| PLANT FATTY ACIDS (MAINLY STEARIC ACID) | STEARIC ACID | 1 |
| GLYCERYL MONO/DISTEARATE / POLYETHYLENE GLYCOL STEARATE MIXTURE (100 OE) | GLYCERYL STEARATE (AND) PEG-100 STEARATE | 1 |
| POLYMETHYLENE WAX HAVING A MELTING POINT OF APPROXIMATELY 64°C | SYNTHETIC WAX | 1 |
| DIISOPROPYL SEBACATE | DIISOPROPYL SEBACATE | 1 |
| DEIONIZED WATER | AQUA | 1 |
| PHENOXY-2 ETHANOL | PHENOXYETHANOL | 0,7 |
| POLY DIMETHYLSILOXANE (VISCOSITY: 350 CST) | DIMETHICONE | 0,5 |
| OCTANE-1,2 DIOL | CAPRYLYL GLYCOL | 0,4 |
| ACRYLIC ACID/STEARYL METHACRYLATE COPOLYMER POLYMERIZED IN AN ETHYL ACETATE/CYCLOHEXANE MIXTURE | ACRYLATES/C10-30 ALKYL ACRYLATE CROSSPOLYMER | 0,25 |
| BAICALIN (PURITY >95%) SCUTELLARIA BAICALENSIS ROOT EXTRACT | SCUTELLARIA BAICALENSIS EXTRACT | 0,2 |
| TRIETHANOLAMINE | TRIETHANOLAMINE | 0,2 |
| XANTHAN: POLYSACCHARIDES: GLUCOSE/MANNOSE/GLUCURONIC ACID (40/30/30) | XANTHAN GUM | 0,2 |
| TRIETHANOLAMINE | TRIETHANOLAMINE | 0,17 |
| ETHYLENE DIAMINE TETRACETIC ACID, DISODIUM SALT, 2 H2O | DISODIUM EDTA | 0,1 |
| TRIETHANOLAMINE | TRIETHANOLAMINE | 0,05 |

### 2.4 FORMULATION D

| **CHEMICAL NAME** | **EU INCI NAME** | **CONCENTRATION** |
|---|---|---|
| MICROBIOLOGICALLY CLEAN DEIONIZED WATER | AQUA | 63,93 |
| GLYCEROL | GLYCERIN | 6 |
| PROPYLENE GLYCOL | PROPYLENE GLYCOL | 6 |
| ISOPROPYL N-LAUROYLSARCOSINATE | ISOPROPYL LAUROYL SARCOSINATE | 5 |
| DENATURED ABSOLUTE ETHYL ALCOHOL | ALCOHOL DENAT. | 3 |
| ISO-HEXADECANE | ISOHEXADECANE | 3 |
| CYCLOHEXA DIMETHYLSILOXANE (VISCOSITY: 8 CST) | CYCLOHEXASILOXANE | 3 |
| TRIAZINE DERIVATIVE (BEMT) = (2,4-BIS((4-(2-ETHYL-HEXYLOXY)-2-HYDROXY)-PHENYL)-6-(4-METHOXYPHENYL)-(1,3,5)-TRIAZINE) | BIS-ETHYLHEXYLOXYPHENOL METHOXYPHENYL TRIAZINE | 1 |
| NON-STABILIZED MONOPOTASSIUM MONO-CETYL PHOSPHATE | POTASSIUM CETYL PHOSPHATE | 1 |
| PLANT FATTY ACIDS (MAINLY STEARIC ACID) | STEARIC ACID | 1 |
| GLYCERYL MONO/DISTEARATE / POLYETHYLENE GLYCOL STEARATE MIXTURE (100 OE) | GLYCERYL STEARATE (AND) PEG-100 STEARATE | 1 |
| POLYMETHYLENE WAX HAVING A MELTING POINT OF APPROXIMATELY 64°C | SYNTHETIC WAX | 1 |
| DIISOPROPYL SEBACATE | DIISOPROPYL SEBACATE | 1 |
| DEIONIZED WATER | AQUA | 1 |
| PHENOXY-2 ETHANOL | PHENOXYETHANOL | 0,7 |
| POLY DIMETHYLSILOXANE (VISCOSITY: 350 CST) | DIMETHICONE | 0,5 |
| OCTANE-1,2 DIOL | CAPRYLYL GLYCOL | 0,4 |
| B-B'CAMPHOSULFONIC ACID [1-4 DIVINYLBENZENE] IN AQUEOUS SOLUTION | TEREPHTHALYLIDENE DICAMPHOR SULFONIC ACID | 0,3 |
| ACRYLIC ACID/STEARYL METHACRYLATE COPOLYMER POLYMERIZED IN AN ETHYL ACETATE/CYCLOHEXANE MIXTURE | ACRYLATES/C10-30 ALKYL ACRYLATE CROSSPOLYMER | 0,25 |
| BAICALIN (PURITY >95%) SCUTELLARIA BAICALENSIS ROOT EXTRACT | SCUTELLARIA BAICALENSIS EXTRACT | 0,2 |
| TRIETHANOLAMINE | TRIETHANOLAMINE | 0,2 |
| XANTHAN: POLYSACCHARIDES: GLUCOSE/MANNOSE/GLUCURONIC ACID (40/30/30) | XANTHAN GUM | 0,2 |
| TRIETHANOLAMINE | TRIETHANOLAMINE | 0,17 |
| ETHYLENE DIAMINE TETRACETIC ACID, DISODIUM SALT, 2 H2O | DISODIUM EDTA | 0,1 |
| TRIETHANOLAMINE | TRIETHANOLAMINE | 0,05 |

### 2.5 FORMULATION E

| **CHEMICAL NAME** | **EU INCI NAME** | **CONCENTRATION** |
|---|---|---|
| MICROBIOLOGICALLY CLEAN DEIONIZED WATER | AQUA | 62,43 |
| GLYCEROL | GLYCERIN | 6 |
| PROPYLENE GLYCOL | PROPYLENE GLYCOL | 6 |
| ISOPROPYL N-LAUROYLSARCOSINATE | ISOPROPYL LAUROYL SARCOSINATE | 5 |
| DENATURED ABSOLUTE ETHYL ALCOHOL | ALCOHOL DENAT. | 3 |
| ISO-HEXADECANE | ISOHEXADECANE | 3 |
| CYCLOHEXA DIMETHYLSILOXANE (VISCOSITY: 8 CST) | CYCLOHEXASILOXANE | 3 |
| 4-TERTIOBUTYL-4'-METHOXYDIBENZOYLMETHANE | BUTYL METHOXYDIBENZOYLMETHANE | 1,5 |
| TRIAZINE DERIVATIVE (BEMT) = (2,4-BIS((4-(2-ETHYL-HEXYLOXY)-2-HYDROXY)-PHENYL)-6-(4-METHOXYPHENYL)-(1,3,5)-TRIAZINE) | BIS-ETHYLHEXYLOXYPHENOL METHOXYPHENYL TRIAZINE | 1 |
| NON-STABILIZED MONOPOTASSIUM MONO-CETYL PHOSPHATE | POTASSIUM CETYL PHOSPHATE | 1 |
| PLANT FATTY ACIDS (MAINLY STEARIC ACID) | STEARIC ACID | 1 |
| GLYCERYL MONO/DISTEARATE / POLYETHYLENE GLYCOL STEARATE MIXTURE (100 OE) | GLYCERYL STEARATE (AND) PEG-100 STEARATE | 1 |
| POLYMETHYLENE WAX HAVING A MELTING POINT OF APPROXIMATELY 64°C | SYNTHETIC WAX | 1 |
| DIISOPROPYL SEBACATE | DIISOPROPYL SEBACATE | 1 |
| DEIONIZED WATER | AQUA | 1 |
| PHENOXY-2 ETHANOL | PHENOXYETHANOL | 0,7 |
| POLY DIMETHYLSILOXANE (VISCOSITY: 350 CST) | DIMETHICONE | 0,5 |
| OCTANE-1,2 DIOL | CAPRYLYL GLYCOL | 0,4 |
| B-B'CAMPHOSULFONIC ACID [1-4 DIVINYLBENZENE] IN AQUEOUS SOLUTION | TEREPHTHALYLIDENE DICAMPHOR SULFONIC ACID | 0,3 |
| ACRYLIC ACID/STEARYL METHACRYLATE COPOLYMER POLYMERIZED IN AN ETHYL ACETATE/CYCLOHEXANE MIXTURE | ACRYLATES/C10-30 ALKYL ACRYLATE CROSSPOLYMER | 0,25 |
| BAICALIN (PURITY >95%) SCUTELLARIA BAICALENSIS ROOT EXTRACT | SCUTELLARIA BAICALENSIS EXTRACT | 0,2 |
| TRIETHANOLAMINE | TRIETHANOLAMINE | 0,2 |
| XANTHAN: POLYSACCHARIDES: GLUCOSE/MANNOSE/GLUCURONIC ACID (40/30/30) | XANTHAN GUM | 0,2 |
| TRIETHANOLAMINE | TRIETHANOLAMINE | 0,17 |
| ETHYLENE DIAMINE TETRACETIC ACID, DISODIUM SALT, 2 H2O | DISODIUM EDTA | 0,1 |
| TRIETHANOLAMINE | TRIETHANOLAMINE | 0,05 |

## Claims

1. A composition for use in a method of photoprotection comprising topical application of said composition, said composition comprising baicalin or an extract containing same, and at least one system filtering UVA radiation comprising at least one bis-resorcinyl triazine derivative.

2. Composition for use according to claim 1, **characterized in that** said bis-resorcinyl triazine derivative is selected from the group consisting of 2,4-bis{[4-(2-ethyl-hexyloxy)-2-hydroxy]-phenyl}-6-(4-methoxy-phenyl)-1,3,5-triazine; 2,4-bis{[4-(tris(trimethyisiloxy) silylpropyloxy)-2-hydroxy]-phenyl}-6-(4-methoxyphenyl)-1,3,5-triazine; 2,4-bis{[4-(1',1',1',3',5',5',5'-heptamethyltrisiloxy-2"-methylpropyloxy)-2-hydroxy]-phenyl}-6-(4-methoxyphenyl)-1,3,5-triazine; and bis-ethylhexyloxyphenol methoxyphenyl triazine, and preferably is bis-ethylhexyloxyphenol methoxyphenyl triazine.

3. Composition for use according to claim 1 or 2, **characterized in that** said composition comprises at least one other UVA filter selected from a dibenzoylmethane derivative, and preferably Avobenzone (1-(4-Methoxyphenyl)-3-(4-*tert*-butylphenyl)propane-1,3-dione), and a compound filtering UVA derived from benzylidene camphor, and preferably Terephthalylidene Dicamphor Sulfonic Acid.

4. Composition for use according to any of claims 1 to 3, **characterized in that** said composition further comprises at least one UVB filter and preferably a β,β'-diphenylacrylate, and more preferably octocrylene.

5. Composition for use according to claim 1, **characterized in that** said system filtering UVA radiation comprises at least Bis-Ethylhexylphenol Methoxyphenyltriazine, Avobenzone, and Terephthalylidene Dicamphor Sulfonic Acid, and **characterized in that** said composition further comprises octocrylene.

6. Composition for use according to any of claims 1 to 5, **characterized in that** said composition further comprises other compounds filtering UVA and/or UVB.

7. Composition for use according to any of claims 1 to 6, **characterized in that** said composition further comprises cosmetically acceptable excipients.

8. Baicalin, or extract containing same, in association with at least one system filtering UVA radiation comprising at least one bis-resorcinyl triazine for use in a method for protecting the skin and/or lips and/or hair against solar radiation.

9. Multi-constituent agent comprising a composition A and a composition B packaged separately;
said composition A comprising, in a cosmetically acceptable medium, at least baicalin or an extract containing same as defined in any one of claims 1 to 8;
said composition B comprising, in a cosmetically acceptable medium, at least one system filtering UVA radiation comprising at least one bis-resorcinyl triazine derivative as defined in any one of claims 1 to 8;
said composition A and said composition B being used in a method of photoprotection applying either simultaneously or sequentially on the surface of the keratin material.

10. Multi-constituent agent for use according to claim 9, where the application of composition A and the application of composition B are sequential and where composition A comprising baicalin is applied first.

11. Multi-constituent agent for use according to claim 10, where the time interval between the applications of A and B varies from 1 hour to 48 hours and more preferentially from 1 hour to 24 hours; filtering composition B being suitable for application immediately prior to exposure to UV particularly to solar radiation.

12. A composition as defined in any one of claims 1 to 8 for use in a method of photoprotection comprising the topical application on human keratin material of said composition for combating or preventing photo-induced premature ageing of a human keratin material.

13. A multi-constituent agent as defined in any one of claims 9 to 11 for use in a method of photoprotection comprising the topical application on human keratin material of said multi-agent for combating or preventing photo-induced premature ageing of a human keratin material.

## Patentansprüche

1. Eine Zusammensetzung zur Verwendung in einem Lichtschutzverfahren, umfassend die topische Anwendung der Zusammensetzung, wobei die Zusammensetzung Baicalin oder einen Extrakt, der dieses enthält, und mindestens ein System, das UVA-Strahlung filtert, enthaltend mindestens ein Bisresorcinyltriazinderivat, umfasst.

2. Zusammensetzung zur Verwendung gemäß Anspruch 1, **dadurch gekennzeichnet, dass** das Bisresorcinyltriazinderivat ausgewählt ist aus der Gruppe bestehend aus 2,4-Bis{[4-(2-ethylhexyloxy)-2-hydroxy]-phenyl}-6-(4-methoxy-phenyl)-1,3,5-triazin; 2,4-Bis{[4-(tris(trimethyisiloxy)silylpropyloxy)-2-hydroxy]-phenyl}-6-(4-methoxyphenyl)-1,3,5-triazin; 2,4-Bis{[4-(1',1',1',3',5',5',5'-heptamethyltrisiloxy-2"-methylpropyloxy)-2-hydroxy]-phenyl}-6-(4-methoxyphenyl)-1,3,5-triazin; und Bisethylhexyloxyphenolmethoxyphenyltriazin und bevorzugt Bisethylhexyloxyphenolmethoxyphenyltriazin ist.

3. Zusammensetzung zur Verwendung gemäß Anspruch 1 oder 2, **dadurch gekennzeichnet, dass** die Zusammensetzung mindestens einen anderen UVA-Filter umfasst, ausgewählt aus einem Dibenzoylmethanderivat und bevorzugt Avobenzon (1-(4-Methoxyphenyl)-3-(4-*tert-*butylphenyl)propan-1,3-dion), und einer von Benzylidenkampfer stammenden Verbindung, die UVA filtert, und bevorzugt Terephthalylidendikampfersulfonsäure.

4. Zusammensetzung zur Verwendung gemäß einem der Ansprüche 1 bis 3, **dadurch gekennzeichnet, dass** die Zusammensetzung ferner mindestens einen UVB-Filter und bevorzugt ein β,'-Diphenylacrylat und stärker bevorzugt Octocrylen umfasst.

5. Zusammensetzung zur Verwendung gemäß Anspruch 1, **dadurch gekennzeichnet, dass** das System, das UVA-Strahlung filtert, mindestens Bisethylhexylphenolmethoxyphenyltriazin, Avobenzon und Terephthalylidendikampfersulfonsäure umfasst und **dadurch gekennzeichnet ist, dass** die Zusammensetzung ferner Octocrylen umfasst.

6. Zusammensetzung zur Verwendung gemäß einem der Ansprüche 1 bis 5, **dadurch gekennzeichnet, dass** die Zusammensetzung ferner weitere Verbindungen umfasst, die UVA und/oder UVB filtern.

7. Zusammensetzung zur Verwendung gemäß einem der Ansprüche 1 bis 6, **dadurch gekennzeichnet, dass** die Zusammensetzung ferner kosmetisch-verträgliche Hilfsstoffe umfasst.

8. Baicalin oder ein Extrakt, der dieses enthält, in Verbindung mit mindestens einem System, das UVA-Strahlung filtert, umfassend mindestens ein Bisresorcinyltriazin zur Verwendung in einem Verfahren zum Schutz der Haut und/oder der Lippen und/oder der Haare gegen Sonnenstrahlung.

9. Mehrkomponentenmittel, umfassend eine Zusammensetzung A und eine Zusammensetzung B, die getrennt verpackt sind;
wobei die Zusammensetzung A in einem kosmetisch-verträglichen Medium mindestens Baicalin oder einen Extrakt, der dieses enthält, wie in einem der Ansprüche 1 bis 8 definiert, umfasst;
wobei die Zusammensetzung B in einem kosmetisch-verträglichen Medium mindestens ein System, das UVA-Strahlung filtert, das mindestens ein Bisresorcinyltriazinderivat, wie in einem der Ansprüche 1 bis 8 definiert, umfasst;
wobei die Zusammensetzung A und die Zusammensetzung B in einem Lichtschutzverfahren verwendet werden, in dem sie entweder gleichzeitig oder nacheinander auf der Oberfläche des Keratinmaterials angewendet werden.

10. Mehrkomponentenmittel zur Verwendung gemäß Anspruch 9, wo die Anwendung der Zusammensetzung A und die Anwendung der Zusammensetzung B nacheinander erfolgen und wo Zusammensetzung A, die Baicalin umfasst, zuerst angewendet wird.

11. Mehrkomponentenmittel zur Verwendung gemäß Anspruch 10, wo das Zeitintervall zwischen den Anwendungen von A und B von 1 Stunde bis 48 Stunden variiert und stärker bevorzugt von 1 Stunde bis 24 Stunden; wobei die Filterungszusammensetzung B zur Anwendung direkt vor der UV-Einwirkung, insbesondere durch Sonnenstrahlung geeignet ist.

12. Eine Zusammensetzung, wie in einem der Ansprüche 1 bis 8 definiert, zur Verwendung in einem Lichtschutzverfahren, umfassend die topische Anwendung der Zusammensetzung auf menschlichem Keratinmaterial, zum Bekämpfen oder Vorbeugen von lichtinduzierter vorzeitiger Alterung eines menschlichen Keratinmaterials.

13. Ein Mehrkomponentenmittel, wie in einem der Ansprüche 9 bis 11 definiert, zur Verwendung in einem Lichtschutzverfahren, umfassend die topische Anwendung des Mehrkomponentenmittels auf menschlichem Keratinmaterial, zum Bekämpfung oder Vorbeugen von lichtinduzierter vorzeitiger Alterung eines menschlichen Keratinmaterials.

## Revendications

1. Composition pour son utilisation dans une méthode de photoprotection comprenant l'application topique de ladite composition, ladite composition comprenant la baïcaline, ou un extrait en contenant, et au moins un système filtrant les radiations UVA comprenant au moins un dérivé de bis-résorcinyl triazine.

2. Composition pour son utilisation selon la revendication 1, **caractérisée en ce que** ledit dérivé de bis-résorcinyl triazine est choisi parmi le groupe constitué de la 2,4-bis {[4-(2-éthyl-hexyloxy)-2-hydroxy]-phényl}-6-(4-méthoxy-phényl)-1,3,5-triazine ; la 2,4-bis {[4-(tris(triméthyisiloxy)silylpropyloxy)-2-hydroxy]-phenyl}-6-(4-méthoxyphényl)-1,3,5-triazine ; la 2,4-bis{[4-(1',1',1',3',5',5',5'-heptaméthyltrisiloxy-2"-méthylpropyloxy)-2-hydroxy]-phényt}-6- (4-méthoxyphényt)-1,3,5-triazine ; et la bis-éthylhexyloxyphénol méthoxyphényl triazine, et de préférence est la bis-éthylhexyloxyphénol méthoxyphényl triazine.

3. Composition pour son utilisation selon la revendication 1 ou 2, **caractérisée en ce qu'**elle comprend en outre au moins un autre filtre UVA choisi parmi un dérivé de dibenzoylméthane, et de préférence l'Avobenzone (1-(4-Methoxyphenyl)-3-(4-*tert-*butylphenyl)propane-1,3-dione), et un composé filtrant les UVA dérivé de benzylidène camphre, et de préférence le Terephtalylidene Dicamphor Sulfonic Acid.

4. Composition pour son utilisation selon l'une quelconque des revendications 1 à 3, **caractérisée en ce qu'**elle comprend en outre au moins un filtre UVB et de préférence un β,β'-diphénylacrylate, et encore de préférence l'octocrylène.

5. Composition pour son utilisation selon la revendication 1, **caractérisée** en ce ledit système filtrant les radiations UVA comprend au moins la Bis-Ethylhexylphenol Methoxyphenyltriazine, l'Avobenzone, et le Terephtalylidene Dicamphor Sulfonic Acid, et **caractérisée en ce que** ladite composition comprend en outre l'octocrylène.

6. Composition pour son utilisation selon l'une quelconque des revendications 1 à 5, **caractérisée en ce qu'**elle comprend d'autres composés filtrant les UVA et/ou les UVB.

7. Composition pour son utilisation selon l'une quelconque des revendications 1 à 6, **caractérisée en ce qu'**elle comprend en outre des excipients cosmétiquement acceptables.

8. Baïcaline, ou d'un extrait en contenant, en association avec au moins un système filtrant les radiations UVA comprenant au moins un dérivé de bis-résorcinyl triazine pour son utilisation dans une méthode pour protéger la peau et/ou les lèvres et/ou les cheveux contre le rayonnement solaire.

9. Agent multi-composants comprenant une composition A et une composition B conditionnées séparément ;
ladite composition A comprenant dans un milieu cosmétiquement acceptable au moins la baïcaline ou un extrait la contenant tels que définis dans les revendications 1 à 8 ; ladite composition B comprenant dans un milieu cosmétiquement acceptable au moins un système filtrant les radiations UVA comprenant au moins un dérivé de bis-résorcinyl triazine tel que défini dans les revendications 1 à 8 ;
ladite composition A et ladite composition B étant utilisées dans une méthode de photoprotection comprenant l'application soit de manière simultanée soit de manière séquentielle sur la surface de la matière kératinique.

10. Agent multi-composants pour son utilisation selon la revendication 9, où l'application de la composition A et l'application de la composition B sont séquentielles et où la composition A comprenant la baicaline est appliquée en premier.

11. Agent multi-composants pour son utilisation selon la revendication 10, où l'intervalle de temps entre les applications de A et de B varie de 1 heure à 48 heures et plus préférentiellement de 1 heure à 24 heures ; la composition B filtrante pouvant être appliquée juste avant l'exposition aux UV en particulier au rayonnement solaire.

12. Composition telle que définie selon l'une quelconque des revendications 1 à 8 pour son utilisation dans une méthode de photoprotection comprenant l'application topique sur une matière kératinique de ladite composition pour lutter contre ou prévenir le vieillissement prématuré photo-induit d'une matière kératinique.

13. Agent multi-composants tel que défini selon l'une quelconque des revendications 9 à 11 pour son utilisation dans une méthode de photoprotection comprenant l'application topique sur une matière kératinique dudit agent multi-composants pour lutter contre ou prévenir le vieillissement prématuré photo-induit d'une matière kératinique.
